# EUROPÄISCHE PATENTANMELDUNG

(11) **EP 1 666 001 A1**
(43) Veröffentlichungstag der Anmeldung: **07.06.2006**
(21) Anmeldenummer: 06006098.5
(22) Anmeldetag: 01.08.2002
(51) Int. Cl.: A61F 2/00

(54) **Inkontinenzband zur Behandlung der Harninkontinenz**

(30) Priorität: 03.08.2001 DE 10138950; 23.03.2002 DE 20204669 U
(62) Teilanmeldung aus: 02754967.4
(71) Anmelder: Aesculap AG & Co. KG, 78532 Tuttlingen/Donau (DE)
(72) Erfinder: Die Erfindernennung liegt noch nicht vor
(74) Vertreter: Patentanwälte Ruff, Wilhelm, Beier, Dauster & Partner

(57) **Zusammenfassung**

Die Erfindung betrifft ein Inkontinenzband (66) zur Behandlung der Harninkontinenz, insbesondere bei der Frau. In einer Ausführungsform ist das Inkontinenzband elastisch und porös ausgebildet. Es weist an seinen Enden jeweils eine Befestigungseinrichtung auf. Weiterhin weist das Inkontinenzband (66) eine es umgebende Hülle (68) auf, welche abziehbar ausgebildet ist und aus zwei zusammenhägenden Teilen (68a,68b) besteht.

## Beschreibung

Die Erfindung betrifft ein Inkontinenzband zur Behandlung der Harninkontinenz, insbesondere bei der Frau.

Zur Behebung der Harninkontinenz sind verschiedene Operationsmethoden und speziell dafür ausgebildete Operationsbestecke und Implantate entwickelt worden. Einige Operationsmethoden erfordern die Öffnung des Bauchraumes, um prothesenartige Gegenstände einzubringen, die die Harnröhre im Bereich des Blasenhalses umschließen oder sie von oben her drücken oder von unten her anheben (WO 00/18319; WO 90/01016; WO 91/00069; US 4,709,690; WO 85/02993).

Bereits in den 60ger Jahren des letzten Jahrhunderts wurde die sogenannte Zügelplastik entwickelt. Bei dieser wird im Bauchraum ein Band fixiert, das unter der Harnröhre hindurchgeführt wird um diese zusammen mit dem Blasenhals etwas anzuheben, was insbesondere in Fällen einer Blasensenkung hilfreich ist. Besonders bewährt hat sich die minimalinvasive Einbringung eines Inkontinenzbandes, das beispielsweise bei weiblichen Patienten durch die Vaginalwand hindurchgeführt wird und als U-förmige Schlinge, die die Harnröhre untergreift, im Bauchraum platziert wird, wobei die freien Enden der Schlinge in der Bauchdecke frei enden. Das Band wird durch einwachsendes Bindegewebe verankert. Solche Bänder und die zugehörigen Instrumente zur Einbringung in den Unterleib sind in den WO 90/03766, WO 96/06567, WO 97/13465 und WO 2001/030246 beschrieben.

Gegenstand der Erfindung ist ein Inkontinenzband zur Behandlung der Harninkontinenz, insbesondere bei der Frau, das in seinen Eigenschaften weitere Anwendungsmöglichkeiten und Vorteile bringt.
Das Inkontinenzband ist flexibel ausgebildet und besteht aus biokompatiblem Material. Es kann ein textiles Band sein, insbesondere ein solches, das beschichtet oder imprägniert ist. Die Beschichtung oder Imprägnierung kann dazu dienen, insbesondere durch das Vorhandensein einer glatten Oberfläche, das Einführen des an sich offenporigen Bandes zu erleichtern. Die Beschichtung oder Imprägnierung kann aus biologisch abbaubarem und/oder resorbierbarem Material bestehen. Das Inkontinenzband kann auch ein mindestens abschnittsweise glattes geschlossenes Band in Form einer Folie sein. Es kann zur Verankerung eine Oberflächenstruktur aufweisen. Bei einer bevorzugten Ausführungsform ist es, in Längsrichtung gesehen, in Material und/oder Struktur verschieden ausgebildet. Es kann in Längsrichtung eine unterschiedliche Form besitzen und/oder Längsabsschnitte mit verschiedenen Eigenschaften aufweisen. Dies ist insbesondere für den mittleren Längsabschnitt von Bedeutung, der zur Anordnung im Bereich der Harnröhre bestimmt ist. In der Regel wird das Band unterhalb der Harnröhre durchgeführt. Es sind aber auch Ausführungen möglich, bei denen das Band oberhalb der Harnröhre angeordnet werden kann, insbesondere wenn es in seiner Dicke verstellbar ist, worauf später noch eingegangen wird.

Da die Harnröhre sehr empfindlich gegenüber Reizungen ist und solche Reizungen unerwünscht starke Gewebewucherungen hervorrufen können, weist das Band in seinem mittleren Bereich vorzugsweise eine glatte Oberfläche auf. Das Band selbst kann mindestens teilweise, insbesondere im mittleren Abschnitt, der zur Anordnung im Bereich der Harnröhre bestimmt ist, aus resorbierbarem Material, insbesondere resorbierbarem Kunststoff, bestehen. Durch vom Inkontinenzband beispielsweise durch eine Oberflächenstrukturierung und/oder Poren ausgeübte mechanische Reize und/oder während der Resorption in Folge eines vermehrten Stoffwechsels bedingte biologische Reize kann eine Vermehrung der Neubildung von Bindegewebe hervorgerufen werden, das eine gewünschte Anhebung bzw. Unterstützung der Harnröhr bewirkt. Dieses unterstützende Bindegewebe kann die Aufgabe des Inkontinenzbandes ersetzen, so daß dies nicht mehr von Nöten ist. Das Band verschwindet mindestens teilweise durch die Resorption. Besteht es vollständig aus biologisch abbaubarem bzw. resorbierbarem Material, dann verschwindet das Band mit der Zeit vollständig. Besteht nur der mittlere Längsabschnitt aus resorbierbarem Material, wogegen die restlichen Abschnitte mindestens teilweise aus nicht resorbierbarem Material bestehen, dann können diese restlichen Abschnitte im Körper verbleiben.

Die resorbierbaren Materialien sind vorzugsweise nicht biologischen Ursprungs, insbesondere resorbierbare Kunststoffe, um Infektionen und immunologische Abwehrreaktionen zu vermeiden. Als Kunststoffe eignen sich die bekannten Polymere und Copolymere von Lactid, Glykolid, Trimethylencarbonat, Dioxanon und ε-Caprolacton. Besonders geeignet ist auch Polyvinylalkohol, dessen Löslichkeit und Resorptionsgeschwindigkeit durch Auswahl des Molekulargewichts, chemische Modifizierung und/oder physikalische Behandlung (Kristallitbildung durch Gefrier-/Tauzyklen) beeinflusst werden können.

Das Band kann auch mit mehreren im wesentlichen parallelen Längsstreifen mit verschiedener Resorbierbarkeit ausgebildet sein, insbesondere mit dem Ziel, Fremdmaterial zu entfernen, sobald es nicht mehr benötigt wird. Unterschiede in Längsrichtung des Inkontinenzbandes, insbesondere im Bezug auf den mittleren Längsabschnitt, können sich auch auf die mechanischen Eigenschaften des Bandes beziehen. So sind die Längskanten des Bandes mindestens im mittleren Längsabschnitt mit Vorteil glatt ausgebildet. In den übrigen Längsabschnitten kann das Band zur besseren Verankerung strukturierte Längskanten und/oder Oberflächen aufweisen. So besitzt der mittlere Längsabschnitt vorzugsweise eine geschlossene, insbesondere glatte Oberfläche. Ferner kann der mittlere Längsabschnitt auch eine größere Breite und/oder größere Dicke besitzen als die übrigen Bereiche des Bandes.

Bei einer besonderen Ausführungsform der Erfindung ist das Band mindestens und insbesondere in seinem mittleren Bereich bzw. Längsabschnitt in der Dicke veränderbar ausgebildet. Das Band kann im mittleren Längsabschnitt doppelwandig, vorzugsweise schlauchförmig ausgebildet sein. Es ist auch möglich diese doppelwandige und insbesondere schlauchförmige Ausbildung am gesamten Band vorzusehen. Das Band besitzt bei einer bevorzugten Ausführungsform im mittleren Längsabschnitt eine mit einem Fluid füllbare Kammer. Durch die flexible Kammerwandung kann sich die Kammer je nach Füllgrad vergrößern oder verkleinern. Es ist auch möglich, die Schlauch- bzw. Kammerwandung mindestens teilweise aus elastisch dehnbarem Material auszubilden. Weiterhin können die Wandungen der mit dem Fluid füllbaren Kammer mindestens teilweise aus mit einer Kanüle durchstechbarem und selbst abdichtendem Material bestehen, insbesondere aus Silikonkautschuk. Dadurch kann die Kammer nachträglich, d.h. nach der Platzierung des Bandes im Körper durch Befüllen oder Absaugen des Fluids in Größe und Form verändert werden. Die Ausführungsform mit der Veränderbarkeit der Dicke ist auch für eine Platzierung oberhalb der Harnröhre geeignet und kann dazu verwendet werden, einen Druck auf die Harnröhre auszuüben, ohne diese zu umschlingen.

Es ist auch möglich, die mit Fluid füllbare Kammer mit einer zumindest zu einem Bandende führenden Fluidleitung zu versehen, durch die Veränderungen des Füllgrades vorgenommen werden können. Die Ausbildung einer solchen Kammer und einer damit verbundenen Fluidleitung kann bei einem Band aus thermoplastischem Folienmaterial durch Verschweißung der Schlauchwandungen mit gewünschter Linienführung vorgenommen werden. Dies ist auch bei thermoplastischen Elastomeren möglich. Bei rein elastomeren Materialien kann bereits bei der Ausbildung des Bandes, insbesondere Schlauches, oder durch nachträgliche Vulkanisation die Formgebung von Kammer und gegebenenfallls Fluidleitung vorgenommen werden.

Das Inkontinenzband kann in klassischer Weise zur Fixierung mit seinen Enden in der Bauchdecke vernäht werden. Bevorzugt ist jedoch eine Selbstverankerung in der Bauchdecke. Hierzu können Bereiche des Bandes, die zur Anordnung in der Bauchdecke bestimmt sind, das sind die Endbereiche des Inkontinenzbandes, eine die Selbstverankerung in der Bauchdecke begünstigende aufgelockerte Oberfläche und/oder poröse Struktur besitzen. Eine solche aufgelockerte Struktur begünstigt den mechanischen Halt im Bindegewebe und außerdem eine zusätzliche Verankerung durch Einwachsen bzw. Durchwachsen von neuem Gewebe.

Das Band ist vorzugsweise so ausgebildet, dass es durch minimalinvasive Operationstechnik in den Bauchraum, insbesondere den Bereich der Harnröhre einziehbar ist, d.h. ohne eine Öffnung des Bauchraumes. Zweckmäßigerweise sind beide Bandenden zur Befestigung an einem zur Platzierung des Bandes im Bauchraum dienenden, insbesondere gebogenen, Schaft ausgebildet. Hierzu können die Bandenden versteift sein. Die Bandenden können schmaler sein als das übrige Band. Ferner kann an den Bandenden eine Lochung vorgesehen sein, worauf auch später bei der Beschreibung weiterer Ausführungsformen noch genauer eingegangen wird. Die Bandenden können auch mit einem Kupplungsstück verbunden sein, das an dem Schaft befestigbar ist.

Bei einer bevorzugten Ausführungsform der Erfindung ist das Band so ausgebildet, dass es verdrehsicher am Schaft befestigbar ist. Dadurch kann beim Einführen des Bandes in den Unterleib eine Orientierung vorgegeben werden. Vorzugsweise ist das Band so am Schaft befestigbar, dass die Bandebene in der Biegungsebene des Schaftes liegt. Das Band kann in voller Breite am Schaft befestigbar sein, dies ist insbesondere dann der Fall, wenn der Schaft selbst ebenfalls flachförmig ausgebildet ist. Bei Schäften mit im wesentlichen kreisförmigem Querschnitt kann die Ausrichtung des Bandes ebenfalls vorgegeben sein, indem das Bandende im Querschnitt U- bzw. V-förmig oder zickzackförmig gefaltet ist, wodurch im weiteren Verlauf die Öffnung des Bandes in seiner Lage vorgegeben ist. So kann das Band in einer vorgegebenen Form an dem Kupplungsstück befestigt sein, das seinerseits verdrehsicher am Schaft befestigbar ist. Das Band kann an seinen Enden schmaler sein als im übrigen Bereich, um die Einführung zu erleichtern. Die Bauchenden können steifer sein als der übrige flexible Bereich.

Bei einer bevorzugten Ausführungsform der Erfindung ist das Band und insbesondere das Kupplungsstück unlösbar am Schaft befestigbar bzw. befestigt. Dadurch kann erreicht werden, dass ein Schaft bei einer Operation nur ein Mal verwendet wird, was aus Gründen der Sterilität bevorzugt ist.

Bei einer weiteren Ausführungsform ist das Band in einer Hülle angeordnet, die abziehbar ausgebildet ist. Die Hülle dient einerseits zum Schutz des Bandes, andererseits kann sie das Einziehen des Bandes in den Unterleib erleichtern, insbesondere dann, wenn das Band an der Oberfläche strukturiert ist bzw. rauhe Stellen aufweist. Die Hülle ist vorzugsweise quergeteilt oder in Querrichtung teilbar, so dass an jedem Bandende ein Hüllenabschnitt nach Implantation abgezogen werden kann. Die Hüllenenden können mit den Bandenden fest verbunden sein, so dass die Hüllenabschitte nach Abschneiden der Bandenden vom Schaft zugänglich sind. Weiterhin können den Bandenden noch Einrichtungen zum Fixieren der Bandenden außerhalb der Bauchdecke zugeordnet sein. Solche Fixierhilfen können dazu dienen, die Bandenden außerhalb der Bauchdecke zu sichern, solange die Hülle über die Enden gezogen wird. Sie können beispielsweise Bandverlängerungen vorübergehende Verdickungen oder mechanische Bremseinrichtungen, wie geschlitzte Gummiknöpfe, sein.

Dem Schaft kann ein Griff zugeordnet sein, an dem der Schaft lösbar verbindbar ist, insbesondere axial und drehgesichert. Der Schaft kann an beiden Enden Einführspitzen zum Durchdringen des Unterleibes aufweisen oder mit solchen verbindbar sein. Weiterhin kann der Schaft an beiden Enden zur lösbaren Verbindung mit dem Griff ausgebildet sein. Ferner kann der Schaft an beiden Enden zur Verbindung mit einem Band ausgebildet sein. Auf diese Weise ist es alternativ möglich, jeweils das eine oder das andere Ende des Schaftes zum Durchdringen des Unterleibes einzusetzen bzw. mit dem Griff oder mit dem Band zu verbinden. Ferner ist es bei einer lösbaren Verbindung zwischen Schaft und Griff bevorzugt, dass das Band nur bei entferntem Griff an dem entsprechenden Schaftende befestigbar ist.

Wie oben bereits erwähnt, kann der Schaft zur Einmalverwendung vorgesehen sein, zumindest während einer Operation. Hierzu ist vorzugsweise jedem Bandende ein eigener Schaft zugeordnet. Es kann eine Sicherungseinrichtung vorgesehen sein, die die Wiederverwendung eines Schaftes während einer Operation verhindert. Hierzu kann das Bandende, insbesondere das Kupplungsstück, unlösbar mit dem Schaft verbindbar sein. Wird der Schaft vom Band getrennt, dann verbleibt das Bandende bzw. das Kupplungsstück beim Trennen des Bandes am Schaft und verhindert dessen Wiederverwendung, weil die Verbindungsmöglichkeit eines weiteren Bandes blockiert ist. Bandende und Kupplungstück sind vorzugsweise als gemeinsame Steckverbindung ausgebildet. Die Verbindung zwischen Schaft und Band kann selbstsperrend erfolgen, so dass die Verbindung ohne Zuhilfenahme besonderer Hilfsmittel nicht mehr lösbar ist.

Der Schaft besitzt normalerweise einen kreisförmigen Querschnitt. Wie bereits erwähnt, kann der Querschnitt des Schaftes in einem zum Einführen in den Körper vorgesehenen Bereich einen von der Kreisform abweichenden Querschnitt aufweisen. Der Schaft kann insbesondere in einer Richtung im Querschnitt um ein mehrfaches größer sein als in der dazu senkrechten Richtung. Diese Verbreiterung kann senkrecht zur Krümmungsebene des Schaftes liegen und liegt vorzugsweise in der Krümmungsebene des Schaftes. Dadurch wird die orientierte Einbringung des Bandes in den Unterleib, insbesondere die orientierte Durchdringung durch die Bauchdecke begünstigt. So kann der Schaft mindestens in einem Längsabschnitt eine Breite besitzen, die in etwa der Breite des daran zu befestigenden Bandes entspricht. Es ist auch möglich, den Schaft über seine gesamte Länge eine Verbreiterung zu verleihen. Ferner ist es möglich und dies ist bei adipösen Patienten bevorzugt, dass der Schaft mindestens abschnittsweise breiter ist als das Band. Etwaige Änderungen der Bandbreite verlaufen vorzugsweise allmählich.

Es ist auch möglich, dass Schaft und Griff unlösbar miteinander verbunden sind, insbesondere einstückig ausgebildet sind. Am freien Schaftende kann dann eine Befestigungseinrichtung für das Band vorgesehen sein. Diese weist vorzugsweise eine Sicherungseinrichtung zur Verhinderung einer unbeabsichtigen Ablösung des Bandes auf.

Es ist bei einer weiteren Ausführungsform auch möglich, den Schaft mit einer flexiblen Hülle zu umgeben, aus der der Schaft, insbesondere beim Durchziehen des Bandes herausziehbar ist. Die flexible Hülle des Schaftes kann aus resorbierbarem Material bestehen und zum vorübergehenden Verbleib im Körper vorgesehen sein. Es ist auch möglich, die flexible Hülle selbst als Inkontinenzband auszubilden. Auf diese Weise kann dann das Band mindestens zum Teil zusammen mit dem Schaft in den Unterleib eingeführt werden.

Bei einer weiteren bevorzugten Ausführungsform eines hüllenaufweisenden Bandes weist mindestens ein Bandende mindestens eine Befestigungseinrichtung zur Befestigung des Bandes an, für dessen Platzierung im Bauchraum vorgesehenen, Applikationshilfen auf. Mit Vorteil ist das Band im Bereich dieser Befestigungseinrichtung verstärkt. Die verstärkte Befestigungseinrichtung ist vorzugsweise flächig ausgebildet und liegt in der Bandebene.

Wie bereits oben gesagt, ist das Band vorzugsweise so ausgebildet, dass es durch minimalinvasive Operationstechnik in den Bauchraum, insbesondere dem Bereich der Harnröhre vaginal oder abdominal einziehbar ist, d.h. ohne eine Öffnung des Bauchraumes. Um das Band möglichst einfach und schonend für den Patienten an der richtigen Stelle im Körper zu platzieren, wird das Band in der Regel unter Zuhilfenahme von Applikationshilfen implantiert. Diese Applikationshilfen werden meist an den Bandenden angebracht und können neben den oben beschriebenen Schäften auch beispielsweise aus Implantationsnadeln oder Schnüren bestehen. Durch die Verstärkungen des erfindungsgemäßen Inkontinenzbandes im Bereich der Befestigungseinrichtung wird ein Ausreißen des Bandes im Bereich der Befestigungseinrichtungen infolge Zugeinwirkung durch die Applikationshilfe verhindert.

Vorzugsweise ist das Inkontinenzband im Bereich der Befestigungseinrichtung durch thermische Verklebung mit einem weiteren Material verstärkt; ("Thermische Verklebung" eines oder mehrerer Materialien mit dem Band bedeutet in diesem Zusammenhang, dass ein oder mehrere Materialien thermisch erweicht oder geschmolzen werden und in die Poren des nicht geschmolzenen Bandes fließen und dort aushärten.) Das Band ist insbesondere in diesem Bereich mit der Hülle des Inkontinenzbandes thermisch verklebt. Durch eine solche thermische Verklebung des Bandes im Bereich der Befestigungseinrichtung mit der Hülle wird auf einfache Art und Weise eine Verstärkung des Bandes in diesem Bereich erreicht. Durch das Miteinbeziehen von weiterem schweißbaren Material kann auch die Flexibilität modifiziert werden.

Um eine zusätzliche Verstärkung des Bandes im Bereich der Befestigungseinrichtung zu erreichen, kann im Bereich der Befestigungseinrichtung zusätzliches thermisch verklebbares Material, insbesondere thermoplastisches Material, miteinbezogen sein. Bei einer bevorzugten Ausführungsform des erfindungsgemäßen Inkontinenzbandes kann zur Verstärkung im Bereich der Befestigungseinrichtung mindestens die Hülle umgeschlagen und verklebt sein. Dieses Umschlagen kann in unterschiedlicher Art und Weise erfolgen. So kann die Hülle beispielsweise an den Ecken umgeschlagen sein, so dass die Verstärkung als im wesentlichen gleichschenkliges Dreieck ausgebildet ist. Dies hat zum beispiel den Vorteil, dass das Inkontinenzband verjüngte Enden besitzt und so leichter und gerichtet durch das Gewebe gezogen werden kann. Bei einer anderen Ausführungsform kann die Hülle an den Endkanten umgeschlagen sein, so dass die Verstärkung als Rechteck oder Quadrat ausgebildet ist. Bei einer weiteren Ausführungsform kann die Hülle an den Ecken und an den Endkanten umgeschlagen sein, was eine besonders hohe Verstärkung im Bereich der Befestigungseinrichtungen mit sich bringt.

Möglich ist auch eine Ausführungsform, bei der zur Verstärkung im Bereich der Befestigungseinrichtungen die Hülle und das Bandende umgeschlagen sind, was eine noch höhere Stabilisierung in diesen Bereichen bewirkt.

Bei einer weiteren bevorzugten Ausführungsform sind die Bandenden abgerundet. Die Formgebung des bzw. der Bandenden kann durch Trennschweißen vorgenommen werden.

Mit Vorteil ist das Band, insbesondere die Hülle, im Bereich der Befestigungseinrichtungen mit mindestens einer weiteren Lage, vorzugsweise mehreren Lagen, thermisch verklebbaren Materials, insbesondere von mindestens einer weiteren Umhüllung, umgeben und mit dieser mindestens teilweise thermisch verklebt. Dies trägt zu einer weiteren Verstärkung im Bereich der Befestigungseinrichtungen des Inkontinenzbandes bei. Mit Vorteil weist mindestens eine der Lagen eine größere Schichtdicke als die Hülle auf. Vorzugsweise bestehen auch diese Lagen thermisch verklebbaren Materials aus thermoplastischem Material.

Die Verklebungsarten mit Hilfe derer die Verstärkungen im Bereich der Befestigungseinrichtungen erreicht werden, können unterschiedlicher Natur sein. In der Regel handelt es sich bei den Verklebungen um Flächenverklebungen, die vorzugsweise flexibel sind. Die Flächenverklebung läßt vorzugsweise eine Biegung um 180° ohne Bruch zu. Je nach Bedarf können die Verklebungen auch Teilflächen- und Punktverklebungen sein, um gewisse Teile flexibler und weicher zu gestalten. Es sind auch Kombinationen der einzelnen Verklebungsarten denkbar. Mit Vorteil können die zu verklebenden Lagen aus Materialien mit verschiedenen Erweichungs- bzw. Schmelzbereich bestehen, so dass bei bestimmten Verklebungen bestimmte Lagen bevorzugt erweichen bzw. schmelzen. So wird bei einer bevorzugten Ausführungsform zunächst bei tiefer Temperatur, bei der zunächst nur die niedrig schmelzenden Lagen, z.B. die Hülle, die aus Polyethylen bestehen kann, erweichen, eine Grundverklebung im Bereich der Befestigungseinrichtungen vorgenommen. Nach dieser Grundverklebung kann im Bereich dieser Grundverklebung eine Teilflächenverklebung bei höherer Temperatur durchgeführt werden. Anschließend kann in bestimmten Abschnitten im Bereich der Teilflächenverklebung eine Punktverklebung bei noch höherer Temperatur erfolgen, z.B. bei einer Temperatur, bei der das Material des Bandes erweicht, das aus Polypropylen, Polyethylentherephthalat, PVA oder anderen nicht absorbierbaren oder absorbierbaren Polymeren wie Polylactiden, Polydioxanonen oder Gemischen aus Glycoliden, Lactiden, Caprolactonen u.ä., bestehen kann. Durch eine solche Kombination von Flächen-, Teilflächen- und Punktverklebungen kann eine Verstärkung gebildet werden, die mittig steifer ist als am Rand. Dadurch werden unterschiedliche Flexibilitäten erreicht. Das Band kann in Form einer strukturierten Folie oder eines Textils aufgebaut sein, und besitzt vorzugsweise einen Erweichungsbereich, der bei einer höheren Temperatur liegt als der der Hülle und gegebenenfalls des Verstärkungsmaterials.

Bei einer besonders bevorzugten Ausführungsform des erfindungsgemäßen Inkontinenzbandes weist die Befestigungseinrichtung mindestens eine Durchgangsöffnung im Bereich der Verstärkung auf. An dieser Durchgangsöffnung kann beispielsweise ein Faden als Applikationshilfe angebracht werden oder ein chirurgisches Instrument direkt eingreifen. Die Durchgangsöffnung kann durch Ausstanzen gebildet sein, vorzugsweise durch Trennschweißen, und besitzt mit Vorteil einen Randbereich, bei dem alle Lagen fest miteinander verbunden sind. Im Gegensatz zu den bekannten Bändern, die unlösbar mit Applikationshilfen verbunden sind, besteht beim erfindungsgemäßen Band der Vorteil, dass die Applikationshilfe in einfacher Weise mit dem Band verbunden und wieder gelöst werden kann und wiederverwendet werden kann. Bei der oben beschriebenen Kombination aus Flächen-, Teilflächen- und Punktverklebungen wird eine Durchgangsöffnung insbesondere im Bereich der Punktverklebungen ausgestanzt. Die Durchgangsöffnung kann in unterschiedlichen Formen, insbesondere eckig ausgebildet sein. Vorzugsweise ist sie rund oder oval ausgebildet.

Vorzugsweise weist das Inkontinenzband an den Enden eine kleinere Dimension als die das Inkontinenzband umgebende Hülle auf.

Bei einer besonders bevorzugten Ausführungsform des erfindungsgemäßen Inkontinenzbandes besteht mindestens die Hülle und gegebenenfalls das weitere Material bzw. die weiteren Lagen aus thermoplastischem Material. Mit Vorteil besteht das Band aus thermoplastischem Material, dessen Schmelzbereich höher liegt als der Schmelzbereich der auf dem Band aufliegenden Lagen. Das Material kann sowohl vom Körper resorbierbar als auch teilweise resorbierbar oder nicht absorbierbar sein.

Weitere Merkmale der Erfindung ergeben sich aus der nachfolgenden Beschreibung von bevorzugten Ausführungsformen der Erfindung zusammen mit der Zeichnung und den Unteransprüchen. Hierbei können die einzelnen Merkmale jeweils für sich alleine oder in Kombination zu mehreren bei einer Ausführungsform der Erfindung verwirklicht sein.

In der Zeichnung zeigen:
- Figur 1:: eine perspektivische Ansicht einer Ausführungsform eines Instruments zur Einführung eines Inkontinenzbandes mit einem gebogenen Schaft und einem Handhabungsgriff für den Schaft in voneinander getrenntem Zustand,
- Figur 2:: die Ausführungsform nach Figur 1 in einer anderen perspektivischen Ansicht,
- Figur 3:: die gleiche Anordnung von Schaft und Handhabungsgriff wie in Figur 1 im Schnitt,
- Figur 4:: einen Längsschnitt mit in den Griff eingeführtem aber noch nicht verriegeltem Schaft,
- Figur 5:: einen Längsschnitt durch Schaft und Griff im verriegelten Zustand,
- Figur 6:: eine perspektivische Ansicht von Schaft und Griff in verriegeltem Zustand,
- Figur 7:: eine Ansicht des Schaftes in der Krümmungsebene des Schaftes,
- Figur 8:: eine Teilansicht des Schaftes mit daran befestigtem Inkontinenzband,
- Figur 9:: ein Schaftende einer anderen Ausführungsform in vergrößertem Zustand das alternativ am einen Griff oder an einem Inkontinenzband befestigbar ist oder auf das eine Spitze aufsteckbar ist.
- Figur 10:: eine Seitenansicht einer anderen Ausführungsform eines Instrumentes zur Einführung eines Inkontinenzbandes,
- Figur 11:: eine gegenüber Figur 10 um 90° gedrehte Ansicht dieser Ausführungsform,
- Figur 12:: eine Ausführungsform eines Inkontinenzbandes und
- Figur 13:: eine andere Ausführungsform eines Inkontinenzbandes.
- Figur 14:: eine Ausführungsform eines erfindungsgemäßen Inkontinenzbandes mit verstärkten Befestigungseinrichtungen,
- Figur 15:: einen stark vergrößerten Längsschnitt entlang der Linie A-A' nach Figur 14,
- Figur 16a:: einen schematischen Querschnitt durch eine weitere Ausführungsform eines erfindungsgemäßen Inkontinenzbandes im nicht verklebten Bereich,
- Figur 16b:: einen schematischen Querschnitt durch das Inkontinenzband nach Figur 16a im Bereich einer Befestigungseinrichtung (verklebter Bereich).

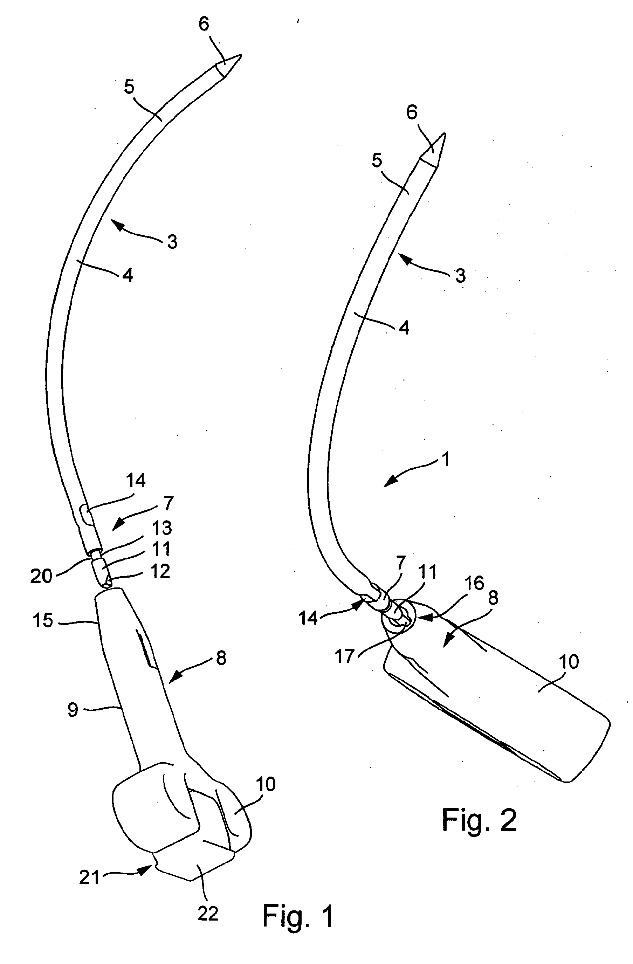

Bei der in den Figuren 1 bis 9 dargestellten Ausführungsform eines Plat-zierungsinstrumentes 1 für ein Inkontinenzband 2 ist ein Schaft zur Einführung des Inkontinenzbandes als gekrümmte massive Nadel 3 aus Edelstahl in Form eines gekrümmten Rundstabes 4 vorgesehen. Die Nadel besitzt an einem Einführende 5 eine kegelförmige Spitze 6. Diese kann auch als Schrägschliff derart ausgebildet sein, daß die Kante des Schliffes auf der Krümmungsinnenseite liegt. Die Nadelspitze ist nicht scharf, damit Verletzungen der Bauchorgane bei der Einführung des Inkontinenzbandes vermieden werden. Die Durchtrennung von Bauchwand und Vaginawand erfolgt ohnehin durch andere Instrumente, wie Skalpelle, so daß die Nadel im wesentlichen nur die Funktion eines Führungsorganes besitzt. Das der Spitze 6 gegenüber liegende Ende ist als Befestigungsende 7 ausgebildet und weist Einrichtungen zur alternativen Befestigung an einem Griff 8 oder an dem Inkontinenzband 2 auf.

Der Griff 8 besteht aus Kunststoff oder Stahl. Er weist einen im wesentlichen als Hohlzylinder ausgebildeten Längsabschnitt 9 auf, der sowohl zur Aufnahme und Befestigung des Nadelendes 7 als auch zum Umfassen mit der Hand dient. Am hinteren Griffende weist der Griff eine querverlaufende Verbreiterung 10 auf, die dem Griff im wesentlichen eine T-Form verleiht und ebenfalls zum Ergreifen dient, und eine feinfühlige Schwenkbewegung der Nadel ermöglicht.

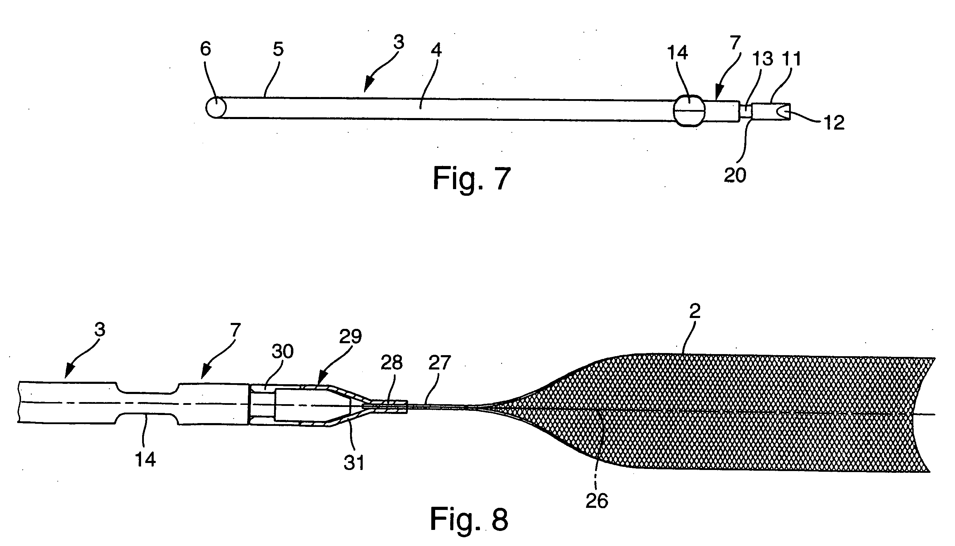

Wie aus Figur 7 ersichtlich, besitzt das Befestigungsende 7 der Nadel 3 einen zylindrischen Abschnitt 11 mit gegenüber dem normalen Durchmesser der Nadel verringertem Durchmesser, der zum freien Ende hin eine schräg verlaufende oder zwei schräg verlaufende gegenüber liegende Abflachungen 12 aufweist und gegen den Rundstab 4 der Nadel 3 durch eine Ringnut 13 abgesetzt ist, die einen zylindrischen Zwischenabschnitt mit noch geringerem Durchmesser bildet. Weiterhin ist das Nadelende im Bereich des vollen Durchmessers mit einer vom runden Querschnitt der Nadel abweichenden Verdrehsicherung, insbesondere in Form einer Flachpressung 14, versehen.

Der Griff 8 ist zur Schnittebene in der T-Form symmetrisch ausgebildet und besitzt im Innern eine Aufnahme zur drehfesten Halterung der Nadel. Hierzu weist er an seinem konisch verjüngten Befestigungsende 15 eine Einschuböffnung 16 auf, deren Innendurchmesser dem normalen Außendurchmesser der Nadel entspricht und die noch zwei seitliche flache längsnutförmige Erweiterungen 17 besitzt, deren Größe der Flachpressung 14 der Nadel entspricht und den verdrehgesicherten Sitz der Nadel in richtiger Ausrichtung zum Griff gewährleistet. Bei dieser Ausrichtung steht die Krümmungsebene der Nadel senkrecht zur Ebene der T-Form des Griffes.

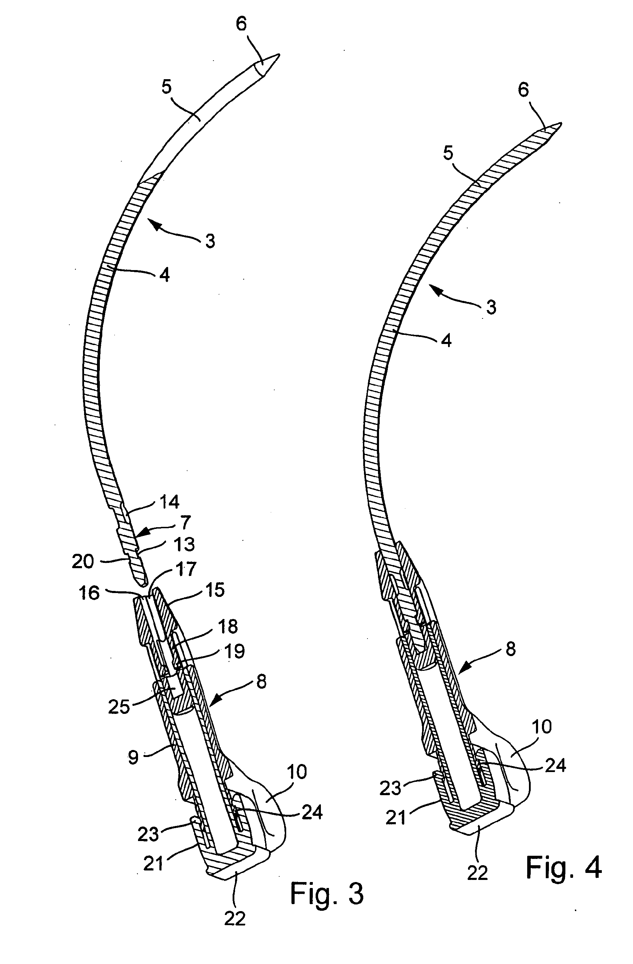

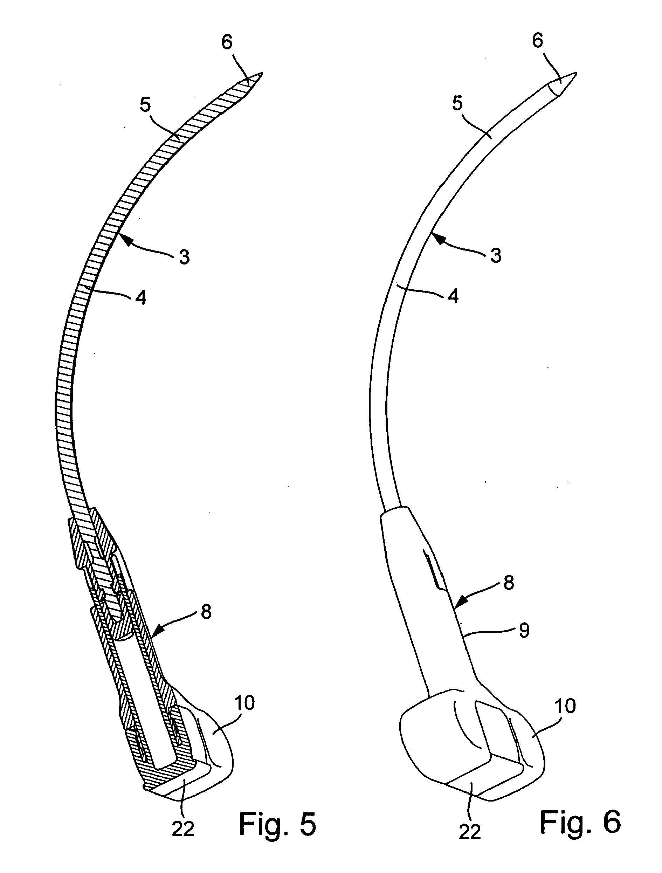

Die Griffwandung des hohlzylinderförmigen Längsabschnittes 9 besitzt zwei zur Ebene der T-Form spiegelbildlich gegenüber liegende fensterartige durch Ausparungen gebildete Materialschwächungen, die als Rastfedern 18 ausgebildet sind. Diese Rastfedern 18 sind längliche Materialfahnen, die an ihrem zur Einschuböffnung des Griffes weisenden Ende einstückig mit dem Material des Längsabschnittes 9 ausgebildet sind und an ihrem freien Ende nach innen weisende Rastnasen 19 besitzen. Die Federn 18 rasten, wie in Figur 4 dargestellt, mit ihren Nasen 19 in die Ringnut 13 des Nadelendes 7 ein, sind jedoch nicht selbstverriegelnd. Vielmehr besitzen die Rastnasen in und gegen die Einschubrichtung weisende Abflachungen und sind beim Einschieben des Nadelendes 7 durch die dazu ausgerichteten Abflachungen 12 des Nadelendes und beim Herausziehen durch die endseitige Ringschulter 20 der Ringnut 13 federnd auseinanderdrückbar. Die Verriegelung der Rastfedern erfolgt mit Hilfe eines im Griff angeordneten längs verschiebbaren hülsenförmigen Schiebers 21, der die Rastfedern 18 in Verriegelungsstellung von außen übergreift (Figur 5) und mit seinem freien zur Betätigung dienenden Ende 22 bündig in der Verbreiterung 10 am Griffende liegt und dort selbstfedernd am Gehäuse des Griffs einrastet. In der entriegelten Stellung (Figur 3 und 4) gibt der Schieber 21 die Rastfedern 18 frei und steht am Griffende über. Rastelemente 23 des Schiebers dienen in entriegelter Stellung zusammen mit entsprechenden Wirkelementen 24 des Griffes auch als Herausfallsicherung für den Schieber 21. Zur Reinigung ist der Griff zerlegbar, d.h. der Schieber 21 kann zur Entfernung über die Wirkelemente des Griffes 8 herausgezogen werden. Im Innern besitzt der Griff auch noch einen Öffnungsabschnitt 25 mit verringertem Innendurchmesser, der dem Außendurchmesser des zylindrischen Abschnittes 11 des Nadelendes 7 entspricht und zur Aufnahme dieses Abschnittes dient. Dadurch wird ein wackelfreier Sitz der Nadel im Griff gewährleistet.

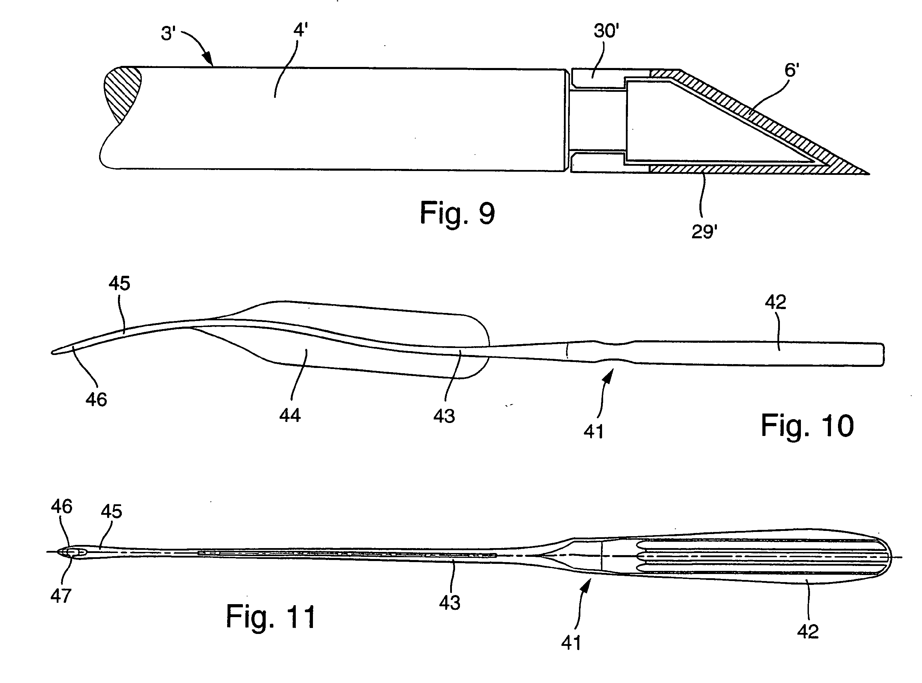

Das Band 2 wird von einem flachen Streifen aus einem textilen Netzmaterial gebildet. Beide Bandenden sind entlang der Mittellinie 26 des Bandes gefaltet, so daß sich ein Endabschnitt 27 mit halber Breite ergibt, dessen Ebene senkrecht zur Bandebene liegt. Diese Endabschnitte 27 sind in ein flachgepresstes Ende 28 einer Befestigungshülse 29 fest eingeklemmt, die als Aufschiebhülse auf das Nadelende 7 zum einrastenden Überschieben des zylindrischen Abschnittes 11 mit verringertem Durchmesser ausgebildet ist. Die Befestigungshülse hat den gleichen Außendurchmesser wie der Rundstab 4 der Nadel. Zur Befestigung am Nadelende 7 ist das freie Ende 30 der Befestigungshülse 29 längs geschlitzt, wobei die Ränder der dadurch gebildeten Fahnen nach innen abgekantet oder verdickt ausgebildet sind und in die Längsnut 13 des Nadelendes einrasten und die Ringschulter 20 formschlüssig hintergreifen. Das Band 2 ist an der Nadel 3 verdrehsicher befestigbar, insbesondere in einer Lage, bei der die Bandebene in der Ebene der Nadelkrümmung liegt. Hierzu wirken die eine (Figur 9) oder beide Abflachungen 12 des zylindrischen Abschitts 11 mit durch die Flachpressung der Befestigungshülse 29 gebildeten Keilflächen 31 zusammen. Auch eine andere Ausbildung der Verdrehsicherung ist möglich.

Bei der dargestellten Ausführungsform der Erfindung kann durch die Bandbefestigung verhindert werden, dass die beiden Enden des Bandes seitlich nacheinander an nur einer Nadel befestigt werden, d.h. es kann verhindert werden, dass dieselbe Nadel bei der Operation zweimal verwendet wird. Hierzu ist vorgesehen, dass die Verbindung der Befestigungshülse 29 am Nadelende 7 unlösbar ist oder nur mit einem hierzu ausgebildeten Werkzeug gelöst werden kann. Dadurch wird die Befestigung des anderen Bandendes am Nadelende 7 unmöglich gemacht und die Wiederverwendung der Nadel bei derselben Operation verhindert. Im Falle einer unlösbaren Verbindung wird die Nadel 3 nach dem Abschneiden des Bandes 2 zusammen mit der Befestigungshülse 29 verworfen. Soll die Nadel 3 wiederverwendbar sein, dann kann die Befestigungshülse 29 vor der Reinigung und Sterilisierung der Nadel mit dem besonderen Werkzeug von der Nadel entfernt werden.

Es kann weiterhin vorgesehen sein, dass die Nadel 3' an beiden Enden Spitzen 6' aufweist, in dem beispielsweise auch das Befestigungsende 7 spitz ausgebildet oder schräg angeschliffen ist. Alternativ kann auch vorgesehen sein, dass Nadelspitzen an der Nadel 3', insbesondere an dem Rundstab 4' befestigbar ausgebildet sind, wie dies in Figur 9 dargestellt ist. Beide Nadelenden können für eine solche Befestigung einer Spitze 6' vorgesehen sein. So können beide Nadelenden wie das Befestigungsende 7' ausgebildet sein. Statt einer Befestigungshülse ist dann eine eine Spitze 6' aufweisende Hülse 29' aufsteckbar. Es kann auch vorgesehen sein, daß die Spitzen wiederabnehmbar ausgebildet sind und nach Abnehmen der Spitze die am Band befestigte Befestigungshülse aufgesteckt wird.

Die in den Figuren 1 bis 9 dargestellten Instrumente sind aufgrund ihrer Variationsmöglichkeiten vielseitig verwendbar und können sowohl für Operationen eingesetzt werden, bei denen der Schaft durch die Bauchwand hindurch von oben nach unten geführt wird oder von unten ausgehend, bei weiblichen Patienten beispielsweise durch die Vaginalwand, nach oben bis zur Bauchdecke. Das Band kann, wie beim Nähen, von der Nadel durchgezogen werden, indem die Nadel durch den Unterleib hindurchgeführt wird und das Band nach sich zieht. Es ist auch möglich, das Band abzuholen, indem beispielsweise die Nadel von der Bauchwand aus nach unten geführt wird, das Band dann an der zugänglichen Nadelspitze befestigt und beim Zurückziehen der Nadel mitgezogen wird.

Demgegenüber besitzt die in den Figuren 10 und 11 dargestellte Ausführungsform eines Instrumentes zum Platzieren eines Inkontinenzbandes weniger Anwendungsmöglichkeiten. Das dort abgebildete Instrument 41 ist jedoch einfacher aufgebaut und weist einige Besonderheiten auf. Das chirurgische Instrument 41 besitzt einen Griff 42 und einen Schaft 43, die einstückig miteinander ausgebildet sind und zweckmäßigerweise aus Edelstahl bestehen. Der Griff 42 ist flach und langgestreckt und kann auch eine andere für chirurgische Instrumente geeignete Form besitzen. Der Schaft 43 verläuft in einer sehr flachen S-Kurve. Er besitzt einen sich im wesentlichen an den Griff 42 anschließenden verbreiterten Abschnitt 44 in Form eines Flachstabes mit parallelen stumpfen Längskanten und einen sich daran anschließenden gekrümmten Abschnitt 45 mit im wesentlichen kreisförmigem Querschnitt, der an seiner Spitze 46 eine seitlich offene Fangnadel 47 zur Befestigung eines Inkontinenzbandes besitzt. Der Übergang vom breiten Abschnitt 44 in den dünnen Abschnitt 45 erfolgt allmählich unter Verjüngung des breiten Abschnittes. Die Ebene des breiten Abschnittes liegt in der Krümmungsebene des gekrümmten Abschnittes 45. Im Vergleich zum Griff 42 verlaufen die Längskanten des verbreiterten Abschnittes 44 leicht nach oben und der sich daran anschließende gekrümmte Abschnitt 45 leicht nach unten. Der Schaft kann aber auch als gekrümmter Stab mit im wesentlichen kreisförmigem Querschnitt ausgebildet sein.

Die Ausführungsform nach Figur 10 ist dazu vorgesehen, daß der Schaft durch die Bauchdecke hindurch in den Bauchraum nach unten geführt wird. Dabei hat der breite Abschnitt 44 die Aufgabe, dem Stichkanal durch die Bauchdecke und das darunter liegende Binde- und Fettgewebe bis zur Vaginalwand eine verbreiterte Form zu verleihen, die in etwa der Form des Inkontinenzbandes entspricht. Die Verbreiterung kann sich, auch im wesentlichen über die gesamte Länge des Schaftes erstrecken. Bei in den Bauchraum eingeführtem Schaft kann das Band an der unten, beispielsweise durch die Vaginalwand, hindurchgeführten Spitze 46 des Schaftes 43 befestigt werden und beim Zurückziehen des Instrumentes mitgezogen werden. Bei zwei Einstichen in die Bauchdecke rechts und links oder vorzugsweise einem zentralen Einstich oberhalb des Schambeines können somit beide Bandenden rechts und links von der Harnröhre hochgezogen werden, so daß sich eine die Harnröhre untergreifende U-förmige Bandführung ergibt.

Die Schaftspitze kann eine lösbare Befestigung des Bandes ermöglichen, insbesondere für eine Befestigung mit Sicherungseinrichtung ausgebildet sein, die ein unbeabsichtigtes Lösen des Bandes vom Schaft verhindert. Bei der dargestellten Ausführungsform ist die Schaftspitze 46 vorzugsweise als sogenannte Reverdinnadel ausgebildet. Diese besitzt eine Öse, die öffenbar und verschließbar ist und im geöffneten Zustand hakenförmig ausgebildet ist. Es ist aber auch möglich, die Befestigungseinrichtung an der Spitze 46 anders zu gestalten, beispielsweise als einfache Öse oder indem die Spitze als abnehmbare Kappe ausgebildet ist und nach Abnehmen der Spitze ein Kupplungsteil freigelegt wird, das mit einem zusammenwirkenden Kupplungsteil des Bandendes verbindbar ist, wie dies im Zusammenhang mit Figur 9 beschrieben wird. Die Öse kann auch durch eine Überwurfmutter verschließbar sein.

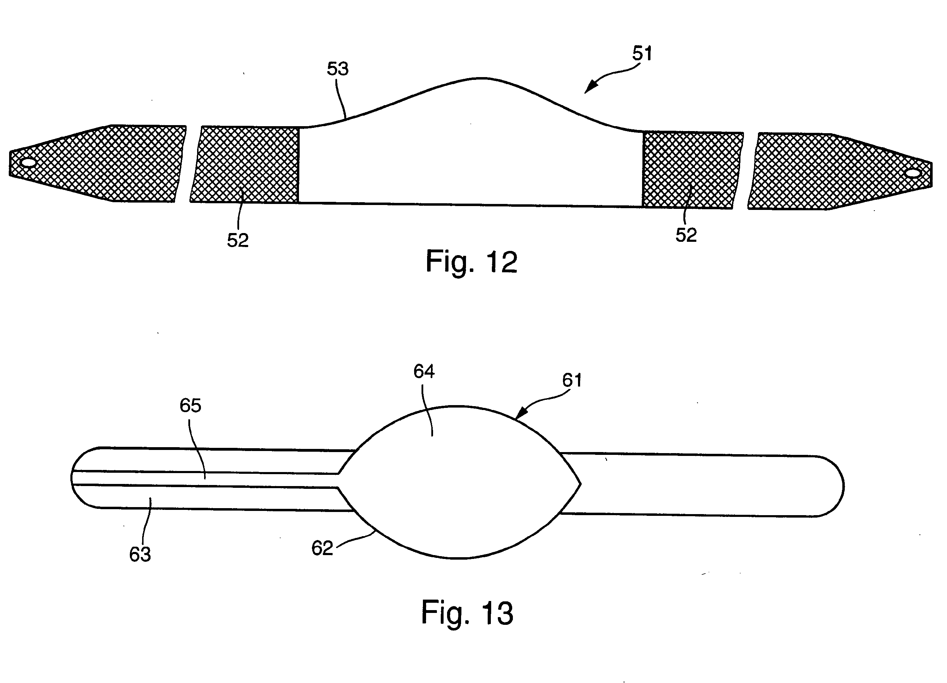

Bei der Ausführungsform eines Inkontinenzbandes nach Figur 12 weist das Inkontinenzbandes 51 voneinander verschiedene Längsabschnitte auf. Zwei Endabschnitte 52 von je etwa 10 bis 20 cm Länge und etwa 2 cm Breite sind als grobmaschige textile Bänder ausgebildet, die an ihren Enden abgerundet sind oder sich V-förmig verjüngen und Löcher oder sonstige Befestigungseinrichtungen zur Befestigung an einem Einführinstrument aufweisen. Ein Mittelabschnitt 53 besteht aus resorbierbarem Kunststoff und ist im Vergleich zu den Endabschnitten 52 auf einer Seite nach Art einer Auswölbung verbreitert. Der Abschnitt besteht aus glattem Folienmaterial und besitzt glatte Ränder. Der Abschnitt ist zum Unterlegen der Harnröhre bestimmt. Durch die glatte Ausbildung und die die Harnröhre schonende Gestaltung wird eine Schädigung der Harnröhre durch unerwünschte Wucherung von neugebildetem Bindegewebe vermieden. Die Einbringung dieses Bandabschnittes als Fremdkörper sorgt für eine ausreichende Gewebeneubildung, die die Harnröhre von unten her unterstützt. Da damit die Funktion des Implantats erfüllt ist, kann insbesondere dieser Bandabschnitt resorbierbar gestaltet sein.

Die Degradationszeit und Resorptionsdauer des Materials des Mittelabschnittes kann in bekannter Weise durch entsprechende Copolymerisation vorbestimmt werden. Als Polymere kommen synthetische Polymere in Frage, insbesondere solche, die durch Polymerisation bzw. Mischpolymerisation von Lactid, Glykolid, Trimethylencarbonat, Dioxanon und ε-Caprolacton erhältlich sind.

Bei der in Figur 13 dargestellten Ausführungsform ist ein Inkontinenzband vorgesehen, das zum dauernden Verbleib im Körper bestimmt ist. Das Inkontinenzband 61 ist wiederum in verschiedene Abschnitte unterteilt, wobei mindestens ein mittlerer Abschnitt 62 als mit einem Fluid, beispielsweise Wasser oder flüssigem Kontrastmittel, füllbare Kammer ausgebildet ist. Hierzu kann der mittlere Abschnitt 62 oder auch das gesamte Band schlauchförmig ausgebildet sein. Hinsichtlich Aufbau und Material gibt es verschiedene Möglichkeiten. Bei der in Figur 13 dargestellten Ausführungsform besteht das Band mindestens im mittleren Abschnitt und mindestens in einem sich daran anschließenden Kanalabschnitt 63 aus thermoplastischem Material. Mit Hilfe von Schweißnähten läßt sich unter Verbindung der beiden Wandungen des Schlauches die Kammer 64 und ein mit dieser kommunizierender Fluidkanal 65 ausbilden, der entlang des Kanalabschnittes 63 verläuft und durch den die Kammer befüllbar ist und durch den das Füllvolumen bei Bedarf nachträglich korrigiert werden kann. Der Fluidkanal 65 kann nach Befüllung beispielsweise durch Verschweißen verschlosssen werden. Er kann unter der Bauchhaut auch durch ein von außen zugängliches Ventil, beispielsweise durch eine durchstechbare Membran zugänglich sein.

Es ist alternativ auch möglich, zumindest die Kammer aus einem Elastomer auszubilden, beispielsweise Silikon, das durchstechbar und selbstabdichtend ist. Dann kann ein besonderer Fluidkanal entfallen. Weiterhin ist es möglich, das ganze Band schlauchförmig auszubilden und lediglich die Schlauchenden zu verschließen. Wesentlich ist, daß auch das Band mit befüllbarer Kammer bandförmig ausgebildet ist, wodurch die minimalinvasive Platzierung des Bandes ermöglicht wird, ohne das eine Eröffnung des Bauchraumes erforderlich ist.

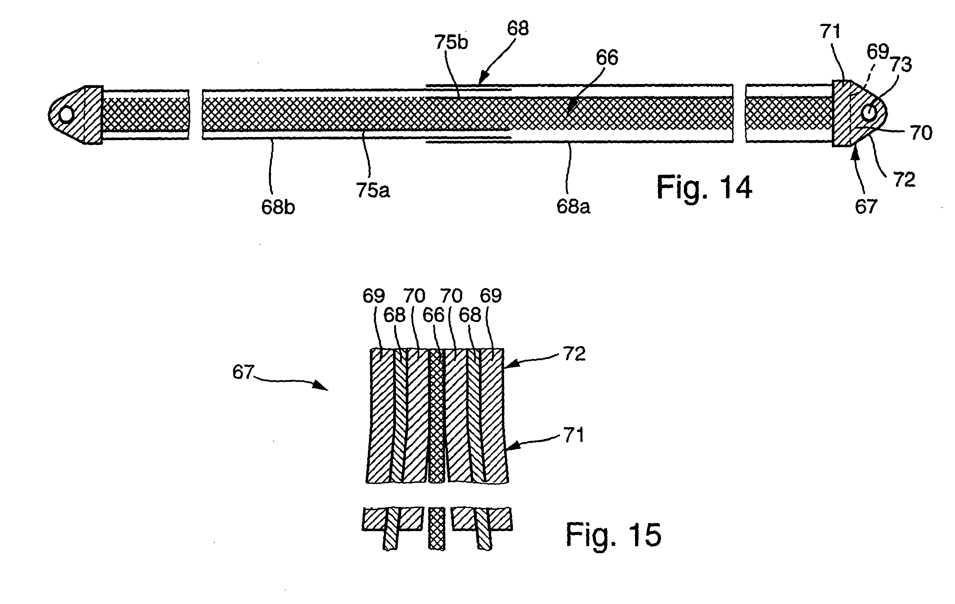

Figur 14 zeigt eine weitere Ausführungsform eines erfindungsgemäßen Inkontinenzbandes 66. Dieses Inkontinenzband ist vorzugsweise elastisch ausgebildet und porös, insbesondere aus Gewirk bestehend, was ein Einwachsen von Gewebe ermöglicht. Dieses Inkontinenzband 66 weist an seinen Enden jeweils eine Befestigungseinrichtung 67 auf. Die Befestigungseinrichtungen weisen die Form von abgestumpften Spitzen auf. Dadurch können sie einfach, gerichtet und sicherer durch das Gewebe geführt werden als dies mit Ecken und Kanten der Fall ist. Die beiden Befestigungseinrichtungen sind gleich ausgebildet. Weiterhin weist das Inkontinenzband 66 eine es umgebende Hülle 68 auf, welche abziehbar ausgebildet ist, indem sie aus zwei nicht zusammenhängenden Teilen (68a, 68b) besteht, welche vorzugsweise in der Mitte des Bandes ineinander gesteckt sind und sich gegenseitig überlappen. Die Hülle kann jedoch auch einstückig ausgebildet sein, wobei sie dann, vorzugsweise in der Bandmitte, eine Perforation zur vereinfachten Trennung in zwei Teile aufweist. Diese Hülle besteht vorzugsweise aus einem thermoplastischen Material, insbesondere aus Polyethylen. Die Hüllen erleichtern die Einführung des Bandes und schützen dieses vor Kontaminierung.

Die Befestigungseinrichtungen 67 sind mehrschichtig aufgebaut. Die oberste Schicht besteht aus einer Umhüllung 69, welche im Bereich der Befestigungseinrichtungen die Hülle 68 umgibt. Auf diese Umhüllung 69 folgt nach innen als zweite Schicht also die Hülle 68. Unter der Hülle 68, also zwischen Hülle 68 und Band 66, befindet sich eine weitere Lage thermisch verklebten Materials 70. Unter dieser Lage 70 befindet sich schließlich das eigentliche Inkontinenzband 66. Das Band 66, die Lage 70, die Hülle 68 sowie die Umhüllung 69 sind an den äußersten Enden des Inkontinenzbandes miteinander verklebt. Ein hinterer Abschnitt 71 der Befestigungseinrichtungen 67 ist verklebungsfrei. Das Band ist in seiner vollen Breite von den Verklebungen und Verstärkungen erfasst, was ein Ausreißen des Gewirks verhindert.
Im verklebten Bereich 72 befindet sich eine Durchgangsöffnung 73, die beim Verkleben ausgestanzt werden kann, so dass die Ränder der Öffnung trenngeklebt sind. Die Stanzung kann verschiedene Formen, wie dreieckig, rechteckig, quadratisch und vorzugsweise rund oder längsoval, aufweisen.

Des weiteren weisen die beiden Hüllenteile 68a und 68b jeweils eine asymmetrisch angeordnete Kennlinie 75a und 75b auf. Diese Kennlinien besitzen vorzugsweise eine gut zu erkennende Färbung. Im Überlappungsbereich der beiden durchsichtigen Hüllenteile 68a und 68b, der vorzugsweise im Bereich der Mitte des Bandes liegt, liegen beide Kennlinien 75a und 75b parallel nebeneinander, so dass der Operateur die Mitte des Bandes erkennen kann.

Alle verwendeten Materialien sind vorzugsweise thermoplastische Materialien. Das Band selbst besteht vorzugsweise aus Polypropylen, die Hülle besteht, wie oben beschrieben, beispielsweise aus Polyethylen. Vorzugsweise ist die Schichtdicke der Umhüllung 69 und der Lage klebbaren Materials 70 höher als die Schichtdicke der Hülle 68. Die Hülle 68 weist vorteilhafterweise eine Schichtdicke von ca. 70 µm auf. Die Schichtdicke der Umhüllung 69 und der Lage klebbaren Materials 70 beträgt vorzugsweise jeweils ca. 200 µm.

Figur 15 zeigt einen stark vergrößerten Längsschnitt durch die Befestigungseinrichtung 67 im Übergangsbereich zwischen verklebtem Bereich 72 und unverklebtem Bereich 71. Im Zentrum der Befestigungseinrichtung 67 befindet sich das Inkontinenzband 66. An das Inkontinenzband 66 schließen sich zwei Lagen klebbaren Materials 70 an. Auf die Lagen klebbaren Materials folgt die Hülle 68. Die Schichtdicke der Hülle 68 ist deutlich geringer als die der Lagen klebbaren Materials 70. Auf seiner Außenseite wird die Befestigungseinrichtung 67 durch die Umhüllung 69 begrenzt. Die Schichtdicke der Umhüllung 69 entspricht in etwa der Schichtdicke der Lagen klebbaren Materials 70. Im Bereich 72 sind die genannten Schichten durch Flächenschweißung miteinander thermisch verklebt. Im Bereich 71 dagegen sind die Schichten untereinander nicht verbunden. Es wäre jedoch möglich, dass die Schichten auch in diesem Bereich verklebt wären, z.B. punktweise. Nach dem Implantieren können die Befestigungseinrichtungen mit den Bandenden abgeschnitten werden und die Hüllenteile 68a und 68b nach beiden Seiten herausgezogen werden. Es ist auch möglich nur eine Hüllenhälfte und die Bandenden mit den Befestigungseinrichtungen zu durchtrennen und die Bandenden zusammen mit den Hüllenteilen nach beiden Seiten herauszuziehen.

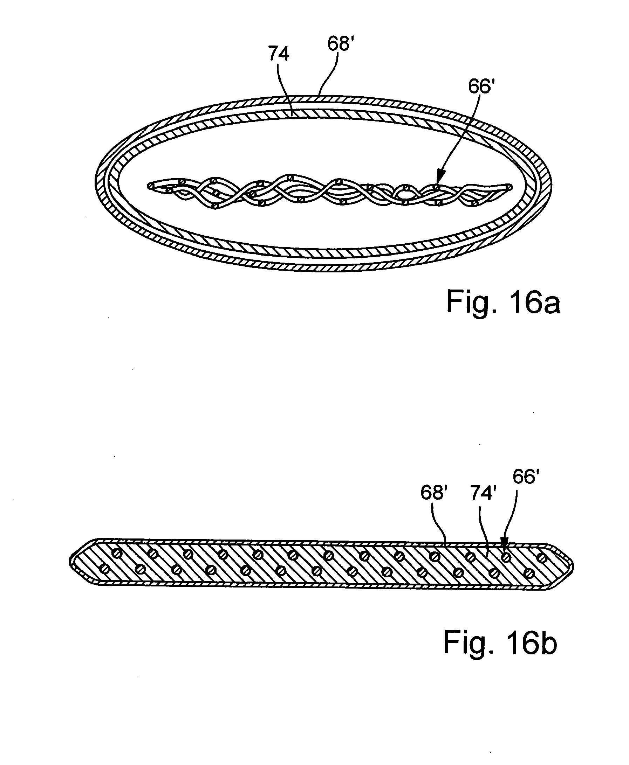

Figur 16a zeigt einen schematischen Querschnitt durch eine weitere Ausführungsform eines erfindungsgemäßen Inkontinenzbandes im noch nicht verklebten Bereich der Bandenden. Das Inkontinenzband 66' besitzt einschließlich Hülle 68' eine Länge von 31cm und eine Breite von 2cm. Das Inkontinenzband 66' ist, wie bereits bei der Beschreibung der Figuren 14 und 15 gesagt, gewirkt und weist Poren auf, was ein Einwachsen von Gewebe ermöglicht. Das Inkontinenzband 66' ist am Bandende von einer Umhüllung 74 umgeben, die eine Länge von 2 bis 8cm besitzt. Die Umhüllung 74 ist wiederum von der Hülle 68', die bereits beim vorhergehenden Ausführungsbeispiel beschrieben wurde, umgeben. Die Umhüllung 74, die zwischen Hülle 68' und dem Bandende eingeordnet ist, umgibt das Inkontinenzband lediglich wenige cm an dessen Längsenden und dient als Verstärkungsmaterial. Die Umhüllung 74 weist einen niedrigeren Schmelzpunkt auf als das Inkontinenzband 66'. Durch diese Eigenschaft kann die Umhüllung 74 als thermoplastische Verklebung zwischen der Hülle und dem Inkontinenzband 66' im Bereich der Befestigungseinrichtungen fungieren (s. Figur 16b) und im sich anschließenden nicht verklebten Bereich eine gewisse Stabilisierung des Bandes bewirken. Die Hülle 68' weist eine Schichtdicke von ca. 70 µm und die Umhüllung 74 eine Schichtdicke von ca. 200 µm auf. Sowohl die Hülle 68' als auch die Umhüllung 74 bestehen aus Polyethylen.

Figur 16b zeigt einen schematischen Querschnitt durch das Inkontinenzband nach Figur 16a im Bereich einer Befestigungseinrichtung (verklebter Bereich). Durch Druck- und Wärmebehandlung des Inkontinenzbandes 66' inklusive seiner Umhüllungen im Bereich der Längsenden wurde erreicht, dass die Umhüllung geschmolzen ist und als thermoplastische Verklebung 74' der Hülle 68' mit dem Inkontinenzband 66 fungiert. Neben der Wirkung als Klebstoff wirkt das Material der Umhüllung 74' im Bereich der Befestigungseinrichtung auch als Füllmaterial zum Ausfüllen der Poren des Inkontinenzbandes 66'. Auch beim vorliegenden Ausführungsbeispiel befindet sich eine Durchgangsöffnung im verklebten Bereich, welche hier jedoch nicht dargestellt ist. Die Gesamtschichtdicke im Bereich der Befestigungseinrichtung beträgt ca. 650 µm.

## Patentansprüche

1. Inkontinenzband (2; 51; 61; 66, 66') zur Behandlung der Harninkontinenz, insbesondere bei der Frau, **dadurch gekennzeichnet, dass** es flexibel ausgebildet ist.

2. Inkontinenzband nach Anspruch 1, **dadurch gekennzeichnet, dass** es in Längsrichtung gesehen in Material und/oder Struktur verschieden ausgebildet ist.

3. Inkontinenzband nach Anspruch 1 oder 2, **dadurch gekennzeichnet, dass** ein mittlerer Längsabschnitt (53; 62) der zur Anordnung im Bereich der Harnröhre bestimmt ist, in Material und/oder Struktur anders aufgebaut ist als das übrige Band (52).

4. Inkontinenzband nach einem der vorhergehenden Ansprüche, **dadurch gekennzeichnet, dass** es mindestens teilweise aus resorbierbarem Material besteht.

5. Inkontinenzband nach einem der vorhergehenden Ansprüche, **dadurch gekennzeichnet, dass** der mittlere, insbesondere nur der mittlere, Längsabschnitt (53) mindestens teilweise aus resorbierbarem Material besteht.

6. Inkontinenzband nach Anspruch 4 oder 5, **dadurch gekennzeichnet, dass** das resorbierbare Material mindestens zum Teil aus Polyvinylalkohol besteht.

7. Inkontinenzband nach einem der vorhergehenden Ansprüche, **dadurch gekennzeichnet, dass** es mindestens im mittleren Längsabschnitt (53; 62) glatte Längskanten aufweist.

8. Inkontinenzband nach einem der vorhergehenden Ansprüche, **dadurch gekennzeichnet, dass** es mindestens im mittleren Längsabschnitt (53; 62) eine glatte, insbesondere geschlossene Oberfläche aufweist.

9. Inkontinenzband nach einem der vorhergehenden Ansprüche, **dadurch gekennzeichnet, dass** es im mittleren Längsabschnitt (53; 62) eine größere Breite und/oder Dicke besitzt als im übrigen Bereich.

10. Inkontinenzband nach einem der vorhergehenden Ansprüche, **dadurch gekennzeichnet, dass** seine Dicke mindestens im mittleren Bereich (62) veränderbar ausgebildet ist.

11. Inkontinenzband nach einem der vorhergehenden Ansprüche, **dadurch gekennzeichnet, dass** es mindestens im mittleren Längsabschnitt (62) doppelwandig, insbesondere schlauchförmig, ausgebildet ist.

12. Inkontinenzband nach einem der vorhergehenden Ansprüche, **dadurch gekennzeichnet, dass** es mindestens im mittleren Längsabschnitt (62) als eine mit einem Fluid füllbare Kammer (64) ausgebildet ist.

13. Inkontinenzband nach Anspruch 12, **dadurch gekennzeichnet, dass** die mit Fluid füllbare Kammer (64) eine zu mindestens einem Bandende führende Fluidleitung (65) besitzt.

14. Inkontinenzband nach Anspruch 12 oder 13, **dadurch gekennzeichnet, dass** Wandungen der mit Fluid füllbaren Kammer und/oder der Fluidleitung (65) mindestens teilweise aus durchstechbarem und selbst abdichtendem Material bestehen.

15. Inkontinenzband nach einem der vorhergehenden Ansprüche, **dadurch gekennzeichnet, dass** es im Bereich seiner Enden eine die Selbstverankerung in der Bauchdecke begünstigende Oberfläche und/oder Struktur besitzt.

16. Inkontinenzband nach einem der vorhergehenden Ansprüche, **dadurch gekennzeichnet, dass** beide Bandenden (52) zur Befestigung an einem zur Platzierung des Bandes im Bauchraum dienenden, insbesondere gebogenen, Schaft (3) ausgebildet sind.

17. Inkontinenzband nach einem der vorhergehenden Ansprüche, **dadurch gekennzeichnet, dass** es an seinen Enden schmaler ist als im übrigen Bereich.

18. Inkontinenzband nach Anspruch 16 oder 17, **dadurch gekennzeichnet, dass** es verdrehsicher am Schaft (3) befestigbar ist.

19. Inkontinenzband nach einem der Ansprüche 16 bis 18, **dadurch gekennzeichnet, dass** es in einer, vorzugsweise in der Biegungsebene des Schaftes (3) liegenden, Ebene ausgerichtet am Schaft (3) befestigbar ist.

20. Inkontinenzband nach einem der Ansprüche 16 bis 19, **dadurch gekennzeichnet, dass** es in einer die Flachform des Bandes vorgegebenen Querschnittsform am Schaft (3) befestigbar ist.

21. Inkontinenzband nach einem der Ansprüche 16 bis 20, **dadurch gekennzeichnet, dass** es an einem mit dem Schaft (3) verbindbaren Kupplungsstück (29) befestigt ist.

22. Inkontinenzband nach einem der Ansprüche 16 bis 21, **dadurch gekennzeichnet, dass** es, insbesondere das Kupplungsstück, am Schaft unlösbar befestigbar ist.

23. Inkontinenzband nach einem der vorhergehenden Ansprüche, **dadurch gekennzeichnet, dass** es in einer vorzugsweise im Mittelabschnitt quergeteilten oder teilbaren Hülle angeordnet ist, die nach Implantation über die Bandenden abziehbar ist.

24. Inkontinenzband nach einem der Ansprüche 16 bis 23, **dadurch gekennzeichnet, dass** ihm mindestens ein Schaft (3) vorzugsweise zwei Schäfte (3) zum Einführen des Bandes (2) in den Unterleib des Patienten zugeordnet sind.

25. Inkontinenzband nach einem der vorhergehenden Ansprüche, **dadurch gekennzeichnet, dass** mindestens ein Bandende mindestens eine Befestigungseinrichtung (67) aufweist.

26. Inkontinenzband nach Anspruch 25, **dadurch gekennzeichnet, dass** das Band im Bereich der Befestigungseinrichtung (67) verstärkt ist.

27. Inkontinenzband nach einem der Ansprüche 25 oder 26, **dadurch gekennzeichnet, dass** das Band im Bereich der Befestigungseinrichtung (67) vorzugsweise in der Bandebene durch thermische Verklebung mit einem weiteren Material verstärkt ist, insbesondere im Bereich der Befestigungseinrichtung (67) mit der Hülle (68) thermisch verklebt ist.

28. Inkontinenzband nach einem der Ansprüche 25 bis 27, **dadurch gekennzeichnet, dass** zur Verstärkung im Bereich der Befestigungseinrichtung (67) zusätzliches thermisch verklebbares Material mit einbezogen ist.

29. Inkontinenzband nach einem der Ansprüche 25 bis 28, **dadurch gekennzeichnet, dass** zur Verstärkung im Bereich der Befestigungseinrichtung (67) mindestens die Hülle (68) umgeschlagen und thermisch verklebt ist.

30. Inkontinenzband nach einem der Ansprüche 25 bis 29, **dadurch gekennzeichnet, dass** das Band, insbesondere die Hülle (68) im Bereich der Befestigungseinrichtung (67) mindestens von einer weiteren Umhüllung (69) umgeben und mit dieser mindestens teilweise thermisch verklebt ist.

31. Inkontinenzband nach einem der Ansprüche 25 bis 30, **dadurch gekennzeichnet, dass** zur Verstärkung im Bereich der Befestigungseinrichtung (67) mindestens eine weitere Lage (70), vorzugsweise mehrere Lagen, thermisch verklebbaren Materials mit einbezogen ist.

32. Inkontinenzband nach Anspruch 31, **dadurch gekennzeichnet, dass** die mindestens eine weitere Lage (70) eine größere Schichtdicke aufweist als die Hülle.

33. Inkontinenzband nach einem der Ansprüche 27 bis 32, **dadurch gekennzeichnet, dass** die thermische Verklebung eine Flächenverklebung ist.

34. Inkontinenzband nach einem der Ansprüche 27 bis 32, **dadurch gekennzeichnet, dass** die thermische Verklebung von Punktverklebungen gebildet wird.

35. Inkontinenzband nach einem der Ansprüche 27 bis 32, **dadurch gekennzeichnet, dass** die thermische Verklebung von Teilflächenverklebungen gebildet wird.

36. Inkontinenzband nach einem der Ansprüche 27 bis 32, **dadurch gekennzeichnet, dass** die thermischen Verklebungen eine Kombination aus Flächenverklebungen, Teilflächenverklebungen und Punktverklebungen sind, wodurch die Befestigungseinrichtung (67) vorzugsweise mittig steifer ist als am Rand und **dadurch** unterschiedliche Flexibilitäten aufweist.

37. Inkontinenzband nach einem der Ansprüche 26 bis 36, **dadurch gekennzeichnet, dass** beide Bandenden Befestigungseinrichtungen (67) mit Verstärkungen aufweisen.

38. Inkontinenzband nach einem der Ansprüche 26 bis 37, **dadurch gekennzeichnet, dass** es im Bereich der Befestigungseinrichtung (67) höchstens dieselbe Breite, insbesondere eine geringere Breite, wie die Hülle (68) außerhalb des verstärkten Bereichs aufweist.

39. Inkontinenzband nach einem der Ansprüche 26 bis 38, **dadurch gekennzeichnet, dass** die Befestigungseinrichtung mindestens eine Durchgangsöffnung (73) aufweist.

40. Inkontinenzband nach einem der Ansprüche 26 bis 39, **dadurch gekennzeichnet, dass** mindestens die Hülle (68) und gegebenenfalls das weitere Material bzw. die weiteren Lagen aus thermoplastischem Material bestehen.

41. Inkontinenzband nach einem der vorhergehenden Ansprüche, **dadurch gekennzeichnet, dass** das Band aus thermoplastischem Material besteht, dessen Schmelzbereich höher liegt als der Schmelzbereich einer auf dem Band aufliegenden Lage.

42. Inkontinenzband nach einem der vorhergehenden Ansprüche, **dadurch gekennzeichnet, dass** das Band aus unterschiedlichen Polymermaterialien besteht, welche in Form einer strukturierten Folie oder eines Textils aufgebaut sind.

43. Inkontinzenzband nach einem der Ansprüche 23 bis 42, **dadurch gekennzeichnet, dass** die im Mittelabschnitt quergeteilte Hülle (68) durchsichtig ist und sich überlappende Hüllenenden aufweist und die beiden Hüllen asymmetrisch angeordnete in Längsrichtung verlaufende Kennlinien (75a und 75b) aufweisen, die im Bereich der beiden sich überlappenden Hüllenenden doppelt vorhanden sind und vorzugsweise parallel zu einander verlaufen.
